# EUROPÄISCHE PATENTANMELDUNG

(11) **EP 3 629 283 A1**
(43) Veröffentlichungstag der Anmeldung: **01.04.2020**
(21) Anmeldenummer: 18197539.2
(22) Anmeldetag: 28.09.2018
(51) Int. Cl.: G06T 5/00, G06T 5/50

(54) **ERZEUGUNG EINES MISCHBILDDATENSATZES**

(71) Anmelder: Siemens Healthcare GmbH, 91052 Erlangen (DE)
(72) Erfinder: HUPFER, Martin, 91052 Erlangen (DE); KÖSTER, Niko, 91077 Neunkirchen am Brand (DE)

(57) **Zusammenfassung**

Die Erfindung betrifft ein Verfahren (10) zur Erzeugung eines Mischbilddatensatzes basierend auf einer energieaufgelösten Aufnahme eines energieauflösenden Röntgendetektors (25) aufweisend die Schritte:
- Erzeugen (11) von einem ersten Bildwert eines ersten Bildelements eines ersten Bilddatensatzes in einem ersten Energiebereich und von einem zweiten Bildwert des ersten Bildelements eines zweiten Bilddatensatzes in einem zweiten Energiebereich,
- Mischen (19) des ersten Bildwerts und des zweiten Bildwerts in Abhängigkeit eines Bildparameters und dabei Erzeugen des Mischbilddatensatzes mit einem Mischbildwert des ersten Bildelements.

## Beschreibung

Die Erfindung betrifft ein Verfahren zur Erzeugung eines Mischbilddatensatzes basierend auf einer energieaufgelösten Aufnahme eines energieauflösenden Röntgendetektors sowie eine Bilderzeugungseinheit dazu.

In der Röntgenbildgebung, beispielsweise in der Computertomographie, der Angiographie oder der Radiographie, können energieauflösende, insbesondere zählende direkt-konvertierende, Röntgendetektoren oder integrierende, insbesondere indirektkonvertierende, Röntgendetektoren verwendet werden.

Die Röntgenstrahlung oder die Photonen können in direktkonvertierenden Röntgendetektoren durch ein geeignetes Konvertermaterial in elektrische Impulse umgewandelt werden. Als Konvertermaterial können beispielsweise CdTe, CdZnTe (CZT), CdZnTeSe, CdTeSe, CdMnTe, InP, TlBr2, HgI2, GaAs oder andere verwendet werden. Die elektrischen Impulse werden von einer Auswerteelektronik, beispielsweise einem integrierten Schaltkreis (Application Specific Integrated Circuit, ASIC), bewertet. In zählenden Röntgendetektoren wird einfallende Röntgenstrahlung durch Zählen der elektrischen Impulse, welche durch die Absorption von Röntgenphotonen im Konvertermaterial ausgelöst werden, gemessen. Die Höhe des elektrischen Impulses ist, insbesondere nach einem Vorverstärker und einem Shaper, in der Regel proportional zur Energie des absorbierten Röntgenphotons. Dadurch kann eine spektrale bzw. eine energieauflösende Information durch den Vergleich der Höhe des elektrischen Pulses mit einem Energieschwellwert extrahiert werden. Durch Vergleich mit mehreren Referenzsignalen bzw. den zugrunde liegenden Energieschwellwerten werden in jedem einzelnen Detektorelement des Röntgendetektors die auftreffenden Photonen in dem entsprechenden Energiebereich oberhalb einer Energieschwelle gezählt.

Diese Referenzsignale bzw. diese Energieschwellwerte werden während eines Verfahrens zur Energiekalibrierung üblicherweise global durch Vorgabe eines einzigen digitalen Wertes (DAC-Wert) pro Energieschwelle für den gesamten Röntgendetektor gesetzt. Jedes Detektorelement kann mehrere individuell einstellbare Energieschwellen mit jeweils einem Energieschwellwert aufweisen. Die Energieschwellwerte können für alle Detektorelemente zumindest teilweise gleich bzw. zumindest teilweise unterschiedlich eingestellt werden, insbesondere nach einer Energiekalibrierung. Alternativ können auch indirekt-konvertierende, energieauflösende Röntgendetektoren, beispielsweise sogenannte Sandwich-Röntgendetektoren, verwendet werden.

Die Ortsauflösung bzw. räumliche Auflösung des Röntgendetektors ist üblicherweise durch das sogenannte Pixel- oder / und Sensordesign, also die Strukturierung des Konverterelements bzw. der Auswerteeinheit in Detektorelemente, gegeben. Durch beispielsweise kleinere Detektorelemente kann die räumliche Auflösung bei der Entwicklung des Röntgendetektors erhöht werden. Die räumliche Auflösung ist damit im Wesentlichen durch die Strukturierung des Konverterelements bzw. des Auswerteeinheit in eine Matrix aufweisend mehrere Detektorelemente festgelegt. Die flächige Größe der Detektorelemente hat zudem Einfluss auf andere Eigenschaften des Detektorelements. Beispielsweise führen in der Regel kleinere Detektorelemente zu einem höheren Pixelrauschen. Ein anderer Nachteil kleinerer Detektorelemente können niedrigere Frame-Raten sein, beispielsweise bedingt durch Bandbreitenlimitierungen bei größere Matrizen mit einer Vielzahl von Detektorelementen. Es können die Daten der Detektorelemente damit bedingt durch die hohen Datenmengen seltener ausgelesen werden.

Die räumliche Auflösung eines Röntgendetektors wird durch seine MTF (Modulation Transfer Function) beschrieben. Für bekannte energieintegrierende Röntgendetektoren ist diese Größe festgelegt und durch das Pixel- und Sensordesign gegeben. Einflussreiche Parameter hinsichtlich der räumlichen Auflösung sind unter anderem die Größe der Detektorelemente, die Sensitivität, die Größe und Form der Diode bzw. die Sensitivität des Konvertermaterials sowie Dicke des Sensor- bzw. Konvertermaterials, beispielsweise CsI, CdTe oder Se. Dies gilt sowohl für direkt-konvertierende wie auch indirektkonvertierende Röntgendetektoren.

Die Erfinder haben erkannt, dass es für manche klinische Anwendungen wünschenswert wäre, retrospektiv, d.h. nach der Datenaufnahme, Einfluss auf die MTF bzw. die räumliche Auflösung nehmen zu können, beispielsweise um mehr Details in feinen Strukturen wie Stents erkennen zu können.

Es ist Aufgabe der Erfindung, ein Verfahren, eine Bilderzeugungseinheit, ein Computerprogrammprodukt, ein computerlesbares Medium und ein medizinisches Gerät anzugeben, welche eine Veränderung, insbesondere eine Verbesserung, der räumlichen Auflösung nach der Datenaufnahme ermöglichen.

Die Aufgabe wird erfindungsgemäß gelöst durch ein Verfahren nach Anspruch 1, eine Bilderzeugungseinheit nach Anspruch 8, ein Computerprogrammprodukt nach Anspruch 9, ein computerlesbares Medium nach Anspruch 10 und ein medizinisches Gerät nach Anspruch 11.

Die Erfindung betrifft ein Verfahren zur Erzeugung eines Mischbilddatensatzes basierend auf einer energieaufgelösten Aufnahme eines energieauflösenden Röntgendetektors aufweisend die Schritte des Erzeugens und des Mischens. Im Schritt des Erzeugens werden der erste Bildwert eines ersten Bildelements eines ersten Bilddatensatzes in einem ersten Energiebereich und der zweite Bildwert des ersten Bildelements eines zweiten Bilddatensatzes in einem zweiten Energiebereich erzeugt. Im Schritt des Mischens werden der erste Bildwert und der zweiten Bildwert in Abhängigkeit eines Bildparameters gemischt und dabei der Mischbilddatensatz mit einem Mischbildwert des ersten Bildelements erzeugt.

Der energieauflösende Röntgendetektor kann insbesondere als zählender Röntgendetektor bevorzugt mit einem direktkonvertierenden Konverterelement ausgebildet sein. Anstatt eines Röntgendetektors kann ein Strahlungs- oder Teilchendetektor verwendet werden. Der Strahlungs- oder Teilchendetektor kann als Röntgendetektor verwendet werden. Der energieauflösende Röntgendetektor kann als Sandwich-Detektor, beispielsweise aufweisend mindestens zwei Konverterschichten im Konverterelement, ausgebildet sein. Die Konverterschichten können direkt- oder indirekt-konvertierend ausgebildet sein. Der Röntgendetektor kann eine Mehrzahl von Detektorelementen aufweisen. Aus den Messwerten der Detektorelemente kann ein Bilddatensatz erzeugt werden. Der Bilddatensatz umfasst Bildwerte. Bei einer zweidimensionalen Röntgenaufnahme kann der Bildwert beispielsweise im einfachsten Fall im Wesentlichen dem Messwert des Röntgendetektors entsprechen. Bei einer dreidimensionalen Röntgenaufnahme kann aus mehreren Aufnahmen und deren Messwerten ein Schichtbild rekonstruiert werden, wobei das Schichtbild den Bilddatensatz bilden kann.

Im Schritt des Erzeugens werden für das erste Bildelement mehrere Bildwerte erzeugt. Der erste Bildwert und der zweite Bildwert umfassen die Bildinformation für einen im Wesentlichen identischen Ort im Bild, jedoch in unterschiedlichen Energiebereichen. Der erste und der zweite Energiebereich können mittels mindestens eines Energieschwellwertes begrenzt werden. Der erste Energiebereich kann beispielsweise mittels einer ersten Energieschwelle zu höheren Energiewerten begrenzt sein. Eine untere Energieschwelle kann oberhalb der Rauschgrenze festgelegt sein und den ersten Energiebereich zu niedrigeren Energiewerten begrenzen. Im ersten Messbereich können Messwerte mit Energiewerten zwischen der unteren Energieschwelle und der ersten Energieschwelle registriert werden. Im zweiten Energiebereich können Messwerte mit Energiewerten oberhalb der ersten Energieschwelle und registriert werden. Die Diskriminierung der Energiebereiche kann einseitig oder zweiseitig erfolgen.

Der Bildparameter kann dem ersten Bildelement oder einer Mehrzahl von Bildelementen des Röntgendetektors zugeordnet sein. Der Bildparameter kann dem ersten Energiebereich oder / und dem zweiten Energiebereich zugeordnet sein. Das erfindungsgemäße Verfahren kann auf mehr als zwei Energiebereiche erweitert werden. Das erfindungsgemäße Verfahren kann für mehrere Bildelemente gleichzeitig oder nacheinander durchgeführt werden. Die Schritte des erfindungsgemäßen Verfahrens können für ein zweites Bildelement wiederholt werden.

Die räumliche Auflösung eines (quanten-)zählenden Röntgendetektors mit kleinen Detektorelementen kann stark vom eingestellten Energieschwellwert abhängen. Bei einem sehr niedrigen Energieschwellwert können Photonen, welche knapp außerhalb des Detektorelements auftreffen, bereits zu einem gezählten Ereignis bzw. einem Messwert führen. Die effektive Apertur des Detektorelements kann damit größer sein als die geometrische Apertur des Detektorelements. Damit kann auch die effektive Apertur des Bildelements größer sein als die geometrische Apertur des Bildelements.

Ist dagegen der Energieschwellwert höher eingestellt, beispielsweise nur knapp unterhalb der Energie der Röntgenphotonen, so können nur Ereignisse gezählt werden, welche das Detektorelement nahe des Zentrums des Detektorelements treffen, da aufgrund der Größe der Ladungswolke in der Regel nur für die zentral auftreffenden Röntgenphotonen die gesamte Energie im Detektorelement deponiert werden kann. Die effektive Apertur kann in diesem Fall also kleiner sein als die geometrische Apertur.

(Quanten-)zählende Röntgendetektoren werten typischerweise mehrere Energieschwellen gleichzeitig aus. Die Erfinder haben erkannt, dass dadurch retrospektiv ein Bilddatensatz mit höherer Ortsauflösung zur Verfügung steht. Da jedoch die Quanteneffizienz eines energieauflösenden, insbesondere zählenden, Röntgendetektors bei höheren Energieschwellwerten geringer sein kann, kann das Erhöhen des Energieschwellwertes dazu führen, dass das Signal-zu-Rausch-Verhältnis in Bereichen des Bilddatensatzes, insbesondere des Bilds, mit wenigen registrierten Ereignissen reduziert wird.

Daher schlagen die Erfinder ein Verfahren vor, in dem die Bilddatensätze der verschiedenen Energiebereiche lokal, beispielsweise in Abhängigkeit von dem Signal und der Bewegung im Bild als Bildparameter, gemischt werden können. Bei hohem Signal bzw. vielen Ereignissen und/oder wenig Bewegung im Bild bzw. zwischen verschiedenen Aufnahmen kann mehr Information von dem höheren Energiebereich bzw. mehreren höheren Energiebereichen in das finale Bild bzw. den Mischbilddatensatz gemischt werden. Die Gewichtung der Ortsinformation aus dem Bilddatensatz mit höherem Energiebereich kann damit erhöht sein. Beispielsweise kann eine lineare Wichtung des ersten Bilddatensatzes und des zweiten Bilddatensatzes durchgeführt werden. Beispielsweise kann eine Zerlegung der Bilddatensätze in verschiedene Bildfrequenzbereiche umfasst sein.

Es können vor dem Mischen des ersten und zweiten Bilddatensatzes eine Filterung oder/und eine Anpassung, beispielsweise basierend auf einer Look-Up-Tabelle, zur Optimierung angewendet werden. Beispielsweise können bereits bekannte Bildalgorithmen, beispielsweise zur Optimierung oder Rekonstruktion von bewegten Bildern, mit dem erfindungsgemäßen Verfahren erweitert und vorteilhaft verbessert werden.

Zusätzlich kann durch Auswerten Bilddatensätze in den verschiedenen Energiebereichen zudem eine Eingrenzung des primären Interaktionspunktes eines Röntgenphotons bzw. Ereignisses innerhalb des Detektorelements bzw. des Röntgendetektors zurückgeschlossen werden. Je niedriger der Energieschwellwert des Energiebereichs ist, desto größer kann auch die Verteilung der möglichen Primärinteraktionsstellen sein. Damit kann beispielsweise mittels mehrerer Energieschwellwerte das registrierte Signal in verschiedene Teile zerlegt werden, mit jeweils unterschiedlich breiten Wahrscheinlichkeitsverteilungen für den Ort der Primärinteraktion.

Die Erfinder schlagen vor, das Mischverhältnis zwischen dem ersten Bilddatensatz und dem zweiten Bilddatensatz insbesondere lokal, d.h. von Bildelement zu Bildelement oder von Bildbereich zu Bildbereich, zu variieren. Beispielsweise kann das Mischverhältnis vom Bildinhalt bzw. von einem Bildparameter abhängig sein.

Vorteilhaft kann die höhere räumliche Auflösung des Bilddatensatzes mit dem höheren Energiebereich für eine Verbesserung der räumlichen Auflösung im Mischbilddatensatz genutzt werden.

Gemäß einem Aspekt der Erfindung weist das Verfahren ferner einen Schritt des Bestimmens auf, wobei der Bildparameter zumindest basierend auf zumindest einem Teilbereich des ersten Bilddatensatzes oder/und des zweiten Bilddatensatzes bestimmt wird. Für unterschiedliche Bildelemente kann ein unterschiedlicher Bildparameter, beispielsweise hinsichtlich Wert oder Art des Bildparameters, bestimmt werden. Der Teilbereich kann mindestens ein Bildelement umfassen. Der Teilbereich kann insbesondere mehrere benachbarte Bildelemente umfassen. Im Schritt des Bestimmens kann der Bildparameter basierend auf dem Bildwert des ersten Bildelements oder den Bildwerten mehrerer Bildelemente umfassend das erste Bildelement des ersten Bilddatensatzes oder / und dem Bildwert des ersten Bildelements oder den Bildwerten mehrerer Bildelemente umfassend das erste Bildelement des zweiten Bilddatensatzes bestimmt werden. Vorteilhaft kann die räumliche Auflösung gezielt verbessert werden.

Gemäß einem Aspekt der Erfindung basiert der Bildparameter auf einer Bildeigenschaft, einer Ortsfrequenzbandeigenschaft oder einer zeitlich veränderlichen Bildeigenschaft gegenüber einer vorherigen Aufnahme. Die Bildeigenschaft kann im Ortsraum oder im Frequenzraum bestimmt werden. Die Bildeigenschaft kann beispielsweise eine Kontrasteigenschaft, eine Intensitätseigenschaft oder eine Information über das Signal-zu-Rausch-Verhältnis umfassen. Die Ortsfrequenzbandeigenschaft kann eine Information über Kanten zwischen beispielsweise verschiedenen Materialien im Untersuchungsobjekt im Bild(-datensatz) oder die Modulationsübertragungsfunktion umfassen. Die zeitlich veränderliche Bildeigenschaft kann aus zwei zeitlich versetzt zueinander aufgenommenen ersten oder / und zweiten Bilddatensätzen, also einer Bildsequenz, bestimmt werden. Beispielsweise kann eine Differenz der Bildwerte zwischen der ersten Aufnahme und der zweiten Aufnahme für ein Bildelement des ersten oder / und zweiten Bilddatensatzes gebildet werden, so dass beispielsweise eine Bewegung im Untersuchungsobjekt oder im Bild festgestellt werden kann. Alternativ können externe Signale als Bildparameter verwendet werden, beispielsweise ein EKG-Signal oder eine Information einer optischen Kameraaufnahme. Übersteigt ein Bildparameter einen geeigneten Schwellwert, so kann das Mischungsverhältnis an die Bildeigenschaft angepasst werden. Vorteilhaft kann die räumliche Auflösung an die Bildeigenschaft angepasst werden. Vorteilhaft kann die räumliche Auflösung in diagnostisch besonders relevanten Teilbereichen des Mischbildes verbessert werden.

Gemäß einem Aspekt der Erfindung weist das Verfahren ferner einen Schritt des Filterns mittels einer Filterfunktion des ersten Bilddatensatzes oder/und des zweiten Bilddatensatzes auf. Der erste oder / und der zweite Bilddatensatz kann beispielsweise mittels einer Fouriertransformation in den Ortsfrequenzraum überführt werden und dort gefiltert werden. Der Schritt des Filterns kann vor oder nach einem Schritt des Bestimmens durchgeführt werden.

Es kann der erste oder / und der zweite Bilddatensatz gefiltert werden. Beispielsweise können im Schritt des Filterns die Inverse der MTF, eine Entrauschung beispielsweise mittels Frequenzbandzerlegung, oder eine Kantenerhaltung im Ortsraum beispielsweise mittels Gradienten aus dem Bilddatensatz des niedrigeren Energiebereichs verwendet werden. Vorteilhaft kann die Bildqualität bzw. der Bildeindruck des Mischbilddatensatzes verbessert werden.

Gemäß einem Aspekt der Erfindung weist das Verfahren ferner einen Schritt des Optimierens der Filterfunktion basierend auf einer Untersuchungsart auf. Die Filterfunktion kann abhängig von den im Aufnahmebereich vorhandenen Materialien im Untersuchungsobjekt, beispielsweise Kontrastmittel wie Jod oder Gewebe (insbesondere Wasser) bzw. Knochen (insbesondere Calcium), gewählt werden. Vorteilhaft kann der erste oder / und der zweite Bilddatensatz an die medizinische Fragestellung angepasst werden. Beispielsweise kann die Filterfunktion für ein Material, eine Untersuchungsart, eine Untersuchungsregion, ein Untersuchungsprogramm oder ein Röntgenspektrum optimiert werden. Das Material kann beispielsweise Jod oder Calcium sein. Das Material kann für ein Objekt im Untersuchungsobjekt typisch sein, beispielsweise ein Draht oder ein Stent. Die Untersuchungsart kann beispielsweise eine Fluoroskopie, Mammographie oder Radiographie sein. Vorteilhaft kann eine optimale Rauschunterdrückung und Kontrastoptimierung im Mischbilddatensatz erreicht werden.

Gemäß einem Aspekt der Erfindung weist der Mischbilddatensatz in mindestens dem ersten Bildelement einen Mischbildwert des ersten Bildelements und in anderen Bildelementen oder in mindestens einem anderen Bildelement den jeweiligen ersten Bildwert oder den jeweiligen zweiten Bildwert auf. Der Mischbildwert entspricht dem aus dem ersten Bildwert und dem zweiten Bildwert gemischten Bildwert im Mischbild. Bei Wiederholen des erfindungsgemäßen Verfahrens für weitere Bildelemente kann zum Beispiel ein Teilbereich mit Mischbildwerten umgeben von ersten Bildwerten oder / und zweiten Bildwerten im Mischbild ausgebildet werden. Der Mischbilddatensatz kann ebenso wie der erste Bilddatensatz als auch der zweite Bilddatensatz mehrere Bildelemente umfassen. Vorteilhaft kann das Bild bzw. der Mischbilddatensatz in einem Teilbereich verbessert werden, wobei in dem anderen Teilbereich die ersten bzw. zweiten Bilddaten erhalten bleiben.

Gemäß einem Aspekt der Erfindung wird das Mischen detektorelementweise im Ortsraum durchgeführt. Die Bildwerte benachbarter Detektorelemente können beispielsweise in den Schritten des Bestimmens, des Filterns oder / und des Optimierens herangezogen werden. Im Schritt des Mischens werden bevorzugt nur die ersten und zweiten Bildwerte des ersten Detektorelements gemischt. Im Schritt des Mischens kann eine Look-Up-Tabelle, eine lineare Gewichtung, eine (nicht-lineare) Funktion, eine Gamma-Funktion oder Logarithmus-Funktion zur Mischung bzw. Gewichtung des ersten Bildwertes und des zweiten Bildwertes verwendet werden. Die Mischung bzw. deren zugrundeliegende Funktion kann auf Messungen, Berechnungen oder maschinellem Lernen basieren. Vorteilhaft kann eine Vermischung der Bildwerte benachbarter Detektorelemente vermieden werden. Vorteilhaft kann die Bildinformation des ersten Detektorelements im Wesentlichen erhalten werden.

Die Erfindung betrifft ferner eine Bilderzeugungseinheit aufweisend Mittel zum Durchführen des erfindungsgemäßen Verfahrens. Die Bilderzeugungseinheit weist eine Erzeugungseinheit zum Erzeugen vom ersten Bilddatensatz und vom zweiten Bilddatensatz, eine Mischeinheit zum Mischen der ersten Bildwerte und der zweiten Bildwerte in Abhängigkeit eines Bildparameters und zum dabei Erzeugen des Mischbilddatensatzes mit einem Mischbildwert des ersten Bildelements auf. Die Bilderzeugungseinheit kann ferner eine Filtereinheit zum Filtern mittels einer Filterfunktion des ersten Bilddatensatzes oder/und des zweiten Bilddatensatzes aufweisen. Die Bilderzeugungseinheit kann ferner eine Bestimmungseinheit zum Bestimmen aufweisen, wobei der Bildparameter zumindest basierend auf zumindest einem Teilbereich des ersten Bilddatensatzes oder/und des zweiten Bilddatensatzes bestimmt wird. Die Bilderzeugungseinheit kann ferner eine Optimierungseinheit zum Optimieren der Filterfunktion basierend auf einer Untersuchungsart aufweisen. Vorteilhaft kann die Bilderzeugungseinheit alle Schritt des erfindungsgemäßen Verfahrens ausführen. In einer alternativen Ausführungsform kann die Bilderzeugungseinheit ein verteiltes System sein.

Die Erfindung betrifft ferner ein Computerprogrammprodukt mit einem Computerprogramm, welches direkt in eine Speichereinrichtung einer Steuereinrichtung eines medizinischen Geräts ladbar ist, mit Programmabschnitten, um alle Schritte des erfindungsgemäßen Verfahrens auszuführen, wenn das Computerprogramm in der Steuereinrichtung des medizinischen Geräts ausgeführt wird. Die Erfindung betrifft ferner ein computerlesbares Medium, auf welchem von einer Rechnereinheit einlesbare und ausführbare Programmabschnitte gespeichert sind, um alle Schritte des erfindungsgemäßen Verfahrens auszuführen, wenn die Programmabschnitte von der Rechnereinheit ausgeführt werden.

Die Erfindung betrifft ferner ein medizinisches Gerät aufweisend eine erfindungsgemäße Bilderzeugungseinheit. Die Vorteile des erfindungsgemäßen Verfahrens können vorteilhaft auf das medizinische Gerät übertragen werden. Das medizinische Gerät kann ein Radiographie-, Fluoroskopie-, Mammographie- oder Computertomographiesystem sein.

Nachfolgend werden Ausführungsbeispiele der Erfindung anhand von Zeichnungen näher erläutert. Hierbei zeigt:
FIG 1 schematisch eine Darstellung des erfindungsgemäßen Verfahrens; und
FIG 2 schematisch eine Darstellung des erfindungsgemäßen medizinischen Geräts.

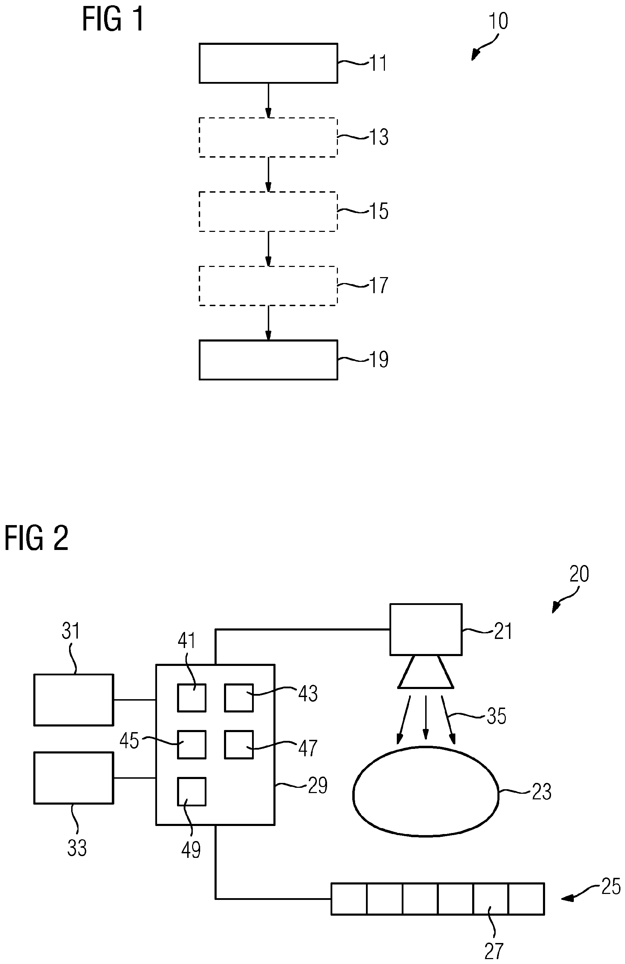

Die Fig. 1 zeigt eine beispielhafte Ausführungsform des erfindungsgemäßen Verfahrens 10. Das Verfahren 10 zur Erzeugung eines Mischbilddatensatzes basierend auf einer energieaufgelösten Aufnahme eines energieauflösenden Röntgendetektors 25 weist die Schritte des Erzeugens 11 und des Mischens 19 auf. Im Schritt des Erzeugens 11 werden ein erster Bildwert eines ersten Bildelements eines ersten Bilddatensatzes in einem ersten Energiebereich und ein zweiter Bildwert des ersten Bildelements eines zweiten Bilddatensatzes in einem zweiten Energiebereich erzeugt. Im Schritt des Mischens 19 werden der erste Bildwert und der zweite Bildwert in Abhängigkeit eines Bildparameters gemischt und dabei ein Mischbilddatensatz mit einem Mischbildwert des ersten Bildelements erzeugt. Der Mischbilddatensatz weist in mindestens dem ersten Bildelement einen Mischbildwert des ersten Bildelements auf. In anderen Bildelementen kann das Mischbild den jeweiligen ersten Bildwert oder den jeweiligen zweiten Bildwert aufweisen. Das Mischen 19 wird detektorelementweise im Ortsraum durchgeführt.

Das Verfahren 10 kann ferner einen Schritt des Bestimmens 13 aufweisen, wobei der Bildparameter zumindest basierend auf zumindest einem Teilbereich des ersten Bilddatensatzes oder/und des zweiten Bilddatensatzes bestimmt wird. Der Bildparameter basiert auf einer Bildeigenschaft, einer Ortsfrequenzbandeigenschaft oder einer zeitlich veränderlichen Bildeigenschaft gegenüber einer vorherigen Aufnahme. Die zeitlich veränderliche Bildeigenschaft kann mittels externer Sensoren bestimmt werden.

Das Verfahren 10 kann ferner einen Schritt des Filterns 17 mittels einer Filterfunktion des ersten Bilddatensatzes oder/und des zweiten Bilddatensatzes aufweisen. Das Verfahren 10 kann ferner einen Schritt des Optimierens 15 der Filterfunktion basierend auf einer Untersuchungsart aufweisen.

Die Fig. 2 zeigt eine beispielhafte Ausführungsform des erfindungsgemäßen medizinischen Geräts 20. Das medizinische Gerät 20 umfasst eine Röntgenquelle 21 und einen gegenüberliegenden energieauflösenden Röntgendetektor 25. Die Röntgenquelle 21 sendet Röntgenstrahlen 35 aus. Das Untersuchungsobjekt 23 ist zwischen der Röntgenquelle 21 und dem Röntgendetektor 25 angeordnet. Der Röntgendetektor 25 weist eine Mehrzahl von Detektorelementen 27 auf. Basierend auf dem Messwert des Detektorelements 27 kann ein Bildwert ermittelt werden. Im einfachsten Fall kann der Bildwert im Wesentlichen dem Messwert entsprechen.

Das medizinische Gerät 20 umfasst ferner eine Bilderzeugungseinheit 29. Die Bilderzeugungseinheit 29 ist mit einer Anzeigeeinheit 31 und einer Eingabeeinheit 33 verbunden. Die Bilderzeugungseinheit 29 umfasst eine Erzeugungseinheit 41 zum Erzeugen vom ersten Bilddatensatz und vom zweiten Bilddatensatz und eine Mischeinheit 43 zum Mischen der ersten Bildwerte und der zweiten Bildwerte in Abhängigkeit eines Bildparameters und dabei Erzeugen des Mischbilddatensatzes mit einem Mischbildwert des ersten Bildelements. Die Bilderzeugungseinheit 29 kann ferner eine Filtereinheit 45, eine Bestimmungseinheit 47 und eine Optimierungseinheit 49 umfassen.

Obwohl die Erfindung im Detail durch das bevorzugte Ausführungsbeispiel näher illustriert wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren (10) zur Erzeugung eines Mischbilddatensatzes basierend auf einer energieaufgelösten Aufnahme eines energieauflösenden Röntgendetektors (25) aufweisend die Schritte:
- Erzeugen (11) von einem ersten Bildwert eines ersten Bildelements eines ersten Bilddatensatzes in einem ersten Energiebereich und von einem zweiten Bildwert des ersten Bildelements eines zweiten Bilddatensatzes in einem zweiten Energiebereich,
- Mischen (19) des ersten Bildwerts und des zweiten Bildwerts in Abhängigkeit eines Bildparameters und dabei Erzeugen des Mischbilddatensatzes mit einem Mischbildwert des ersten Bildelements.

2. Verfahren (10) nach Anspruch 1, ferner aufweisend einen Schritt des Bestimmens (13), wobei der Bildparameter zumindest basierend auf zumindest einem Teilbereich des ersten Bilddatensatzes oder/und des zweiten Bilddatensatzes bestimmt wird.

3. Verfahren (10) nach einem der vorangehenden Ansprüche, wobei der Bildparameter auf einer Bildeigenschaft, einer Ortsfrequenzbandeigenschaft oder einer zeitlich veränderlichen Bildeigenschaft gegenüber einer vorherigen Aufnahme basiert.

4. Verfahren (10) nach einem der vorangehenden Ansprüche, ferner aufweisend einen Schritt des Filterns (17) mittels einer Filterfunktion des ersten Bilddatensatzes oder/und des zweiten Bilddatensatzes

5. Verfahren (10) nach Anspruch 4, ferner aufweisend einen Schritt des Optimierens (15) der Filterfunktion basierend auf einer Untersuchungsart.

6. Verfahren (10) nach einem der vorangehenden Ansprüche, wobei der Mischbilddatensatz in mindestens dem ersten Bildelement einen Mischbildwert des ersten Bildelements und in anderen Bildelementen den jeweiligen ersten Bildwert oder den jeweiligen zweiten Bildwert aufweist.

7. Verfahren (10) nach einem der vorangehenden Ansprüche, wobei das Mischen (19) detektorelementweise im Ortsraum (durchgeführt wird.

8. Bilderzeugungseinheit (29) aufweisend Mittel zum Durchführen des Verfahrens gemäß einem der Ansprüche 1 bis 7, aufweisend:
- eine Erzeugungseinheit (41) zum Erzeugen vom ersten Bilddatensatz und vom zweiten Bilddatensatz,
- eine Mischeinheit (43) zum Mischen der ersten Bildwerte und der zweiten Bildwerte in Abhängigkeit eines Bildparameters und dabei Erzeugen des Mischbilddatensatzes mit einem Mischbildwert des ersten Bildelements.

9. Computerprogrammprodukt mit einem Computerprogramm, welches direkt in eine Speichereinrichtung einer Steuereinrichtung eines medizinischen Geräts ladbar ist, mit Programmabschnitten, um alle Schritte eines Verfahrens nach einem der Ansprüche 1 bis 7 auszuführen, wenn das Computerprogramm in der Steuereinrichtung des medizinischen Geräts ausgeführt wird.

10. Computerlesbares Medium, auf welchem von einer Rechnereinheit einlesbare und ausführbare Programmabschnitte gespeichert sind, um alle Schritte eines Verfahrens nach einem der Ansprüche 1 bis 7 auszuführen, wenn die Programmabschnitte von der Bilderzeugungseinheit (29) ausgeführt werden.

11. Medizinisches Gerät (20) aufweisend eine Bilderzeugungseinheit (29) nach Anspruch 8.
